(19) Europäisches Patentamt
European Patent Office
Office européen des brevets

(11) EP 1 646 673 B1

(12) # EUROPÄISCHE PATENTSCHRIFT

(45) Veröffentlichungstag und Bekanntmachung des Hinweises auf die Patenterteilung:
**14.03.2007 Patentblatt 2007/11**

(21) Anmeldenummer: **04740460.3**

(22) Anmeldetag: **30.06.2004**

(51) Int Cl.:
***C08G 65/332*** *(2006.01)* ***C08G 65/26*** *(2006.01)*
***C07C 69/54*** *(2006.01)* ***C07C 67/08*** *(2006.01)*

(86) Internationale Anmeldenummer:
**PCT/EP2004/007078**

(87) Internationale Veröffentlichungsnummer:
**WO 2005/005514 (20.01.2005 Gazette 2005/03)**

(54) **(METH)ACRYLS UREESTER MONOALKOXILIERTER POLYOLE UND DEREN HE RSTELLUNG**

(METH)ACRYLIC ACID ESTERS OF MONOALKOXYLATED POLYOLS, AND PRODUCTION THEREOF

(METH)ACRYLATES DE POLYOLS MONOALCOXYLES ET LEUR PRODUCTION

(84) Benannte Vertragsstaaten:
**AT BE BG CH CY CZ DE DK EE ES FI FR GB GR HU IE IT LI LU MC NL PL PT RO SE SI SK TR**

(30) Priorität: **10.07.2003 DE 10331450**

(43) Veröffentlichungstag der Anmeldung:
**19.04.2006 Patentblatt 2006/16**

(73) Patentinhaber: **BASF Aktiengesellschaft**
**67056 Ludwigshafen (DE)**

(72) Erfinder:
- **HERMELING, Dieter**
  **67459 Böhl-Iggelheim (DE)**
- **DANIEL, Thomas**
  **67165 Waldsee (DE)**
- **ELLIOTT, Mark**
  **67063 Ludwigshafen (DE)**
- **RIEGEL, Ulrich**
  **66849 Landstuhl (DE)**
- **DIETSCHE, Frank**
  **69198 Schriesheim (DE)**
- **SCHWALM, Reinhold**
  **67157 Wachenheim (DE)**

(56) Entgegenhaltungen:
**EP-A- 0 322 110** **WO-A-93/21237**
**DE-A- 2 913 218**

- **DATABASE WPI Section Ch, Week 199425 Derwent Publications Ltd., London, GB; Class A25, AN 1994-206551 XP002301485 & JP 06 145341 A (NIPPON OILS & FATS CO LTD) 24. Mai 1994 (1994-05-24)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 646 673 B1

## Beschreibung

Die vorliegende Erfindung betrifft neue (Meth)acrylsäureester monoalkoxilierter Polyole, ein Verfahren zu deren Herstellung sowie deren Verwendung zur Herstellung von vernetzten quellbaren hydrogelbildenden Polymeren und vernetzte quellbare hydrogelbildende Polymere.

Der Begriff (Meth)acrylsäure bzw. (Meth)acrylsäureester steht für Methacrylsäure und Acrylsäure bzw. Methacrylsäureester und Acrylsäureester.

Quellbare hydrogelbildende Polymere, sogenannte Superabsorber (Super-Absorbing Polymers, SAP), sind aus dem Stand der Technik bekannt.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Gute Transporteigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Innenvernetzung erreicht, wodurch allerdings die Retention des Produktes verringert wird. Eine elegante Methode zur weiteren Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Durch die Oberflächennachvernetzung steigt die Vemetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein.

Die Patentanmeldung EP-A-0 238 050 offenbart als mögliche Innenvernetzer für Superabsorber Trimethylolpropantriacrylat, mindestens zweifach mit Acrylsäure oder Methacrylsäure verestertes Glycerin, zweifach bzw. dreifach mit Acrylsäure oder Methacrylsäure veresterte Anlagerungsprodukte von Ethylenoxid und/oder Propylenoxid an Trimethylolpropan.

Kommerziell erhältlich ist beispielsweise von Sartomer (Exton, PA, USA) unter den angegebenen Handelsnamen Trimethylolpropantriacrylat (SR 351), dreimal einfach ethoxiliertes Trimethylolpropantriacrylat (SR 454), dreimal zweifach ethoxiliertes Trimethylolpropantriacrylat (SR 499), dreimal dreifach ethoxiliertes Trimethylolpropantriacrylat (SR 502), dreimal fünffach ethoxiliertes Trimethylolpropantriacrylat (SR 9035) und insgesamt 20-fach ethoxiliertes Trimethylolpropantriacrylat (SR 415). Propoxilierte Trimethylolpropantriacrylate sind unter den Handelsnamen SR 492 (dreimal einfach propoxiliertes Trimethylolpropantriacrylat) und CD 501 (dreimal zweifach propoxiliertes Trimethylolpropantriacrylat) erhältlich.

Aus WO 93/21237 sind (Meth)acrylate von alkoxilierten mehrwertigen $C_2$ - $C_{10}$-Kohlenwasserstoffen als Vernetzer bekannt. Verwendet wurden Trimethylolpropanvernetzer, die SR 351, SR 454, SR 502, SR 9035 und SR 415 der Firma Sartomer entsprechen. Diese Vernetzer weisen 0, 3, 9, 15 oder 20 Ethylenoxid-Einheiten pro Molekül Trimethylolpropantriacrylat auf. Vorteilhaft sind laut WO 93/21237 2 bis 7 Ethylenoxid-Einheiten pro Kette im Trimethylolpropantriacrylat, insbesondere 4 bis 6 EO-Einheiten pro Kette im Trimethylolpropantriacrylat.

Ethoxilierte Trimethylolpropantri(meth)acrylate werden in der Patentliteratur immer wieder als Innenvernetzer erwähnt, wobei nur die kommerziell von Sartomer erhältlichen Trimethylolpropantriacrylat-Derivate verwendet werden, so z.B. in WO 98/47951 dreimal einfach ethoxiliertes Trimethylolpropantriacrylat, in WO 01/41818 Sartomer® SR 9035 als sogenanntes hoch ethoxiliertes Trimethylolpropantriacrylat (HeTMPTA) und in WO 01/56625 Sartomer® SR 9035 und Sartomer® SR 492.

Es bestand die Aufgabe weitere Verbindungen zur Verfügung zu stellen, die als Radikalvernetzer für Superabsorber verwendet werden können.

Der vorliegenden Erfindung liegt weiter die Aufgabe zugrunde vernetzte, wasserquellbare Polymere mit einem ausgewogenen Eigenschaftsprofil hinsichtlich Absorptionsvermögen, Gelfestigkeit, Aufnahmegeschwindigkeit und extrahierbaren Anteilen anzugeben, die sich außerdem vorteilhaft in einem kontinuierlichen Verfahren herstellen lassen.

Unter Vernetzung wird in dieser Schrift, wenn nicht anders erwähnt, die Gelvernetzung, Innenvernetzung oder Quervernetzung von linearem oder schwach vernetzten Polymer verstanden. Diese Vernetzung kann über radikalische

oder kationische Polymerisationsmechanismen oder andere, beispielsweise Michael-Addition, Ver- oder Umesterungsmechanismen erfolgen, bevorzugt durch radikalische Polymerisation.

Vernetzte quellbare hydrogelbildende Polymere sind bevorzugt solche mit einer Absorption von 0,9 Gew.-%iger Kochsalzlösung von mindestens dem 10-fachen Eigengewicht bezogen auf das eingesetzte Polymer, bevorzugt dem 20-fachen Eigengewicht. Diese Absorption wird bevorzugt auch unter einem Druck beispielsweise von 0,7 psi erreicht.

Es wurde nun gefunden, dass diese Aufgaben unter Verwendung neuer Vernetzer gelöst werden.

Demgemäß betrifft die Erfindung (Meth)acrylsäureester monoalkoxilierter Polyole der allgemeinen Formel I

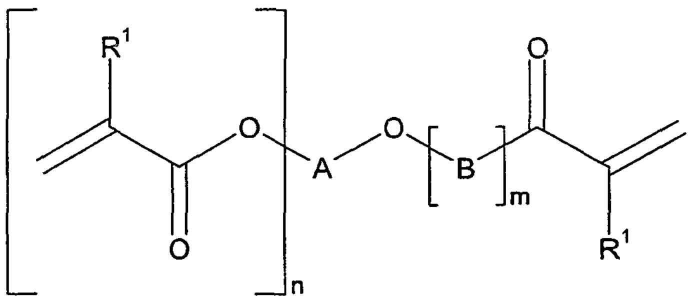

(I)

in der

R1 Wasserstoff oder Methyl,

n eine ganze Zahl von 2 bis 5,

m eine ganze Zahl von 1 bis 100,

A $C_3$- bis $C_{20}$-Alk(n+1)yl oder $C_3$- bis $C_{20}$-Heteroalk (n+1) yl

bedeuten, und
B für gleiche oder verschiedene Reste, ausgewählt aus der Gruppe

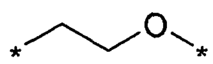

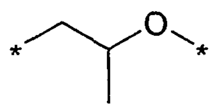

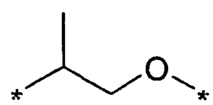

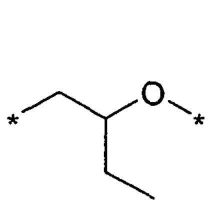

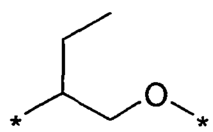

steht und mit * die Verknüpfungspositionen gekennzeichnet sind.

Bevorzugt sind (Meth)acrylsäureester monoalkoxilierter Polyole der allgemeinen Formel I, in der

$R^1$ Wasserstoff oder Methyl,
n 2 oder 3,
m eine ganze Zahl von 2 bis 50,
A $C_3$- bis $C_{10}$-Alk(n+1)yl

bedeuten, und
B für gleiche oder verschiedene Reste, ausgewählt aus der Gruppe

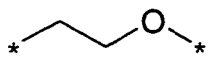

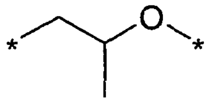

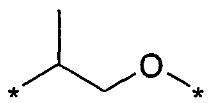

steht und mit * die Verknüpfungspositionen gekennzeichnet sind.
Besonders bevorzugt sind (Meth)acrylsäureester monoalkoxilierter Polyole der allgemeinen Formel I, in der

$R^1$ Wasserstoff oder Methyl,

n 2,

m eine ganze Zahl von 3 bis 30,

A $C_3$- bis $C_6$-Alk(n+1)yl

bedeuten, und
B

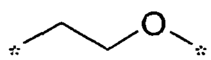

ist und mit * die Verknüpfungspositionen gekennzeichnet sind.
Ganz besonders bevorzugt sind (Meth)acrylsäureester monoalkoxilierter Glycerine.
Die erfindungsgemäßen (Meth)acrylsäureester monoalkoxilierter Polyole lassen sich beispielsweise aus einem Alkohol der allgemeinen Formel II

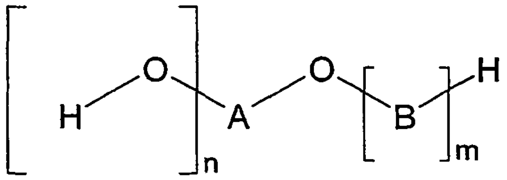

(II)

in der

m, n, A und B die obengenannten Bedeutungen haben,
durch Umsetzung mit (Meth)acrylsäure, Umesterung mit einem (Meth)acrylsäureester sowie durch Acidolyse mit (Meth) acrylsäurechlorid oder (Meth)acrylsäureanhydrid herstellen. Die Umsetzung mit (Meth)acrylsäure ist bevorzugt.

Die Herstellung von (Meth)acrylsäureestern durch säurekatalysierte Veresterung von (Meth)acrylsäure mit Alkanolen ist allgemein bekannt, siehe beispielsweise Ullmann's Enzyclopedia of Industrial Chemistry, Vol. A1, Seiten 162 bis 169, VCH 1985. Der Bildung des Esters aus (Meth)acrylsäure und Alkanol liegt bekanntlich eine Gleichgewichtsreaktion zugrunde. Um wirtschaftliche Umsätze zu erzielen, wird in der Regel ein Einsatzstoff im Überschuss eingesetzt und/oder das gebildete Veresterungswasser aus dem Gleichgewicht entfernt. Um die Abtrennung des Wassers zu beschleunigen und zu erleichtern, wird in der Regel ein organisches Lösungsmittel zugesetzt, das mit Wasser nicht mischbar ist und/oder ein Azeotrop mit Wasser bildet. Als Lösungsmittel werden häufig aliphatische, cycloaliphatische und/oder aromatische Kohlenwasserstoffe verwendet, wie Pentane, Hexane, Heptane, Cyclohexan oder Toluol, siehe beispielsweise DE-A-20 50 678, DE-A-29 13 218, US-4,053,504, US-2,917,538 und EP-A-0 618 187.

Als Alkohole einsetzbar sind monoalkoxilierter Polyole, die durch Umsetzung eines partiell geschützten Polyols mit mindestens einem Alkylenoxid erhältlich sind.

Vorzugsweise werden die Schutzgruppen erst unmittelbar vor der Veresterung entfernt. Geeignete Katalysatoren für die Hydrolyse sind die obengenannten Veresterungskatalysatoren, vorzugsweise Schwefelsäure.

Geeignete partiell geschützte Polyole sind beispielsweise Methylidenglycerin (4-Hydroxymethyl-1,3-dioxolan), Ethylidenglycerin (4-Hydroxymethyl-2-methyl-1,3-dioxolan), Isopropylidenglycerin (4-Hydroxymethyl-2,2-dimethyl-1,3-dioxolan), sek.-Butylidenglycerin (2-Ethyl-4-Hydroxymethyl-2-methyl-1,3-dioxolan), Glycerin-1,2-divinylether, Glycerin-1,3-divinylether, Methylidentrimethylolpropan (5-Ethyl-5-hydroxymethyl-1,3-dioxan), Ethylidentrimethylolpropan (5-Ethyl-5-hydroxymethyl-2-methyl-1,3-dioxan), Isopropylidentrimethylolpropan (5-Ethyl-5-hydroxymethyl-2,2-dimethyl-1,3-dioxan), sek.-Butylidentrimethylolpropan (2,5-Diethyl-5-hydroxymethyl-2-methyl-1,3-dioxan), Trimethylolpropan-divinylether und Pentaerythrit-trivinylether. Bevorzugt sind Isopropylidenglycerin und Isopropylidentrimethylolpropan und ganz besonders bevorzugt ist Isopropylidentrimethylolpropan.

Geeignete Alkylenoxide sind beispielsweise Ethylenoxid, Propylenoxid und/oder Butylenoxid.

Die Alkylenoxidkette kann bevorzugt aus Ethylenoxid-, Propylenoxid- und/oder Butylenoxideinheiten zusammengesetzt sein. Eine solche Kette kann sich aus einer Spezies eines Alkylenoxides oder aus einem Gemisch von Alkylenoxiden zusammensetzen. Wird ein Gemisch verwendet, können die unterschiedlichen Alkylenoxideinheiten statistisch oder als Block oder Blöcke einzelner Spezies vorliegen. Bevorzugt ist als Alkylenoxid Ethylenoxid, Propylenoxid oder ein Gemisch daraus, besonders bevorzugt ist Ethylenoxid oder ein Gemisch aus Ethylenoxid und Propylenoxid und ganz besonders bevorzugt Ethylenoxid.

Die bevorzugte Anzahl der Alkylenoxideinheiten in der Kette beträgt von 1 bis 100, bevorzugt von 2 bis 50, besonders bevorzugt von 3 bis 30, ganz besonders bevorzugt von 4 bis 20.

Die angegebenen Alkoxylierungsgrade beziehen sich dabei jeweils auf den mittleren Alkoxylierungsgrad.

Die Umsetzung der partiell geschützten Polyole mit einem Alkylenoxid ist dem Fachmann an sich bekannt. Mögliche Durchführungsformen finden sich in Houben-Weyl, Methoden der Organischen Chemie, 4. Auflage, 1963, Thieme Verlag Stuttgart, Band 14/2, Seiten 440 bis 444.

Werden gemischt monoalkoxilierte Polyole verwendet, so können die darin enthaltenen unterschiedlichen Alkoxygruppen zueinander im molaren Verhältnis von beispielsweise 0,05 - 20 : 1, bevorzugt 0,1 - 10 : 1, und besonders bevorzugt 0,2 - 5 : 1, stehen.

An die Viskosität der erfindungsgemäß einsetzbaren monoalkoxilierten Polyole werden keine besonderen Ansprüche gestellt, außer dass sie bei einer Temperatur bis zu ca. 80°C problemlos pumpbar sein sollten, bevorzugt sollten sie eine Viskosität unter 1000 mPas aufweisen, bevorzugt unter 800 mPas, und ganz besonders bevorzugt unter 500 mPas.

Die Herstellung der partiell geschützten Polyole ist dem Fachmann an sich bekannt. Beispielsweise beschreibt die Patentanmeldung DE-A-196 47 395 die Synthese von Isopropylidenglycerin, sek.-Butylidenglycerin und Isopropylidentrimethylolpropan.

Als saurer Veresterungskatalysator wird bevorzugt Schwefelsäure eingesetzt. Andere brauchbare Veresterungskatalysatoren sind organische Sulfonsäuren, z.B. Methansulfonsäure, Benzolsulfonsäure, p-Toluolsulfonsäure oder Dodecylbenzolsulfonsäure und/oder saure Ionenaustauscher. Der Veresterungskatalysator kommt im Allgemeinen in einer Menge von 0,1 bis 10 Gew.-%, vorzugsweise 0,5 bis 5 Gew.-%, bezogen auf (Meth)acrylsäure und monoalkoxiliertes Polyol, zur Anwendung.

Als Polymerisationsinhibitoren einsetzbar sind beispielsweise Phenole wie Alkylphenole, wie beispielsweise o-, m- oder p-Kresol (Methylphenol), 2-tert.-Butyl-4-methylphenol, 2,6-Di-tert.-Butyl-4-methylphenol, 2-tert.-Butylphenol, 4-tert.-Butylphenol, 2,4-Di-tert.-butylphenol, Hydrochinon, Brenzkatechin (1,2-Dihydroxybenzol), Aminophenole, wie beispielsweise para-Aminophenol, Nitrosophenole, wie beispielsweise para-Nitrosophenol, p-Nitroso-o-kresol, Alkoxyphenole, wie beispielsweise 2-Methoxyphenol (Guajacol, Brenzkatechinmonomethylether), 2-Ethoxyphenol, 2-Isopropoxyphenol, 4-Methoxyphenol (Hydrochinonmonomethylether), Chinone und Hydrochinone, wie beispielsweise Hydrochinon

oder Hydrochinonmonomethylether, 2,5-Di-tert.-Butylhydrochinon, Benzochinon, N-Oxyle, wie beispielsweise 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, aromatische Amine, wie beispielsweise Phenylendiamine, N,N-Diphenylamin, N-Nitroso-diphenylamin, Nitrosodiethylanilin, N,N'-Dialkyl-para-phenylendiamin, wobei die Alkylreste gleich oder verschieden sein können und jeweils unabhängig voneinander aus 1 bis 4 Kohlenstoffatomen bestehen und geradkettig oder verzweigt sein können, wie beispielsweise N,N'-Di-iso-butyl-p-phenylendiamin, N,N'-Di-iso-propyl-p-phenylendiamin, Hydroxylamine, wie beispielsweise N,N-Diethylhydroxylamin, Harnstoffderivate, wie beispielsweise Harnstoff oder Thioharnstoff, phosphorhaltige Verbindungen, wie beispielsweise Triphenylphosphin, Triphenylphosphit, hypophosphorige Säure oder Triethylphosphit, schwefelhaltige Verbindungen, wie beispielsweise Diphenylsulfid, Phenothiazin oder Metallsalze, wie beispielsweise Kupfer-, Mangan-, Cer-, Nickel-, Chrom-chlorid, -dithiocarbamat, -sulfat, -salicylat oder -acetat oder Gemische davon.

Bevorzugt sind die genannten Phenole und Chinone, besonders bevorzugt sind Hydrochinon, Hydrochinonmonomethylether, 2-tert.-Butyl-4-methylphenol, 2,6-Di-tert.-butyl-4-methylphenol, 2,4-Di-tert.-butylphenol, Triphenylphosphit, hypophosphorige Säure, $CuCl_2$ und Guajacol.

Besonders bevorzugt sind Hydrochinonmonomethylether, Hydrochinon, und Alkylphenole, gegebenenfalls in Kombination mit Triphenylphosphit und/oder hypophosphoriger Säure.

Zur weiteren Unterstützung der Stabilisierung kann ein sauerstoffhaltiges Gas, bevorzugt Luft oder ein Gemisch aus Luft und Stickstoff (Magerluft) anwesend sein. Besonders bevorzugt enthält das sauerstoffhaltige Gas weniger als 10 Vol.% Sauerstoff, ganz besonders bevorzugt 4 bis 6 Vol.-% Sauerstoff.

Unter den aufgeführten Stabilisatoren sind solche bevorzugt, die aerob sind, d.h. solche, die zur Entfaltung ihrer vollen Inhibitorwirkung die Anwesenheit von Sauerstoff erfordern.

Selbstverständlich können bei der Veresterung auch Lösungsmittel eingesetzt werden besonders solche, die sich zur azeotropen Entfernung des Wassers eignen, vor allem aliphatische, cycloaliphatische und aromatische Kohlenwasserstoffe oder Gemische davon.

Vorzugsweise kommen n-Pentan, n-Hexan, n-Heptan, Cyclohexan, Methylcyclohexan, Benzol, Toluol oder Xylol zur Anwendung. Besonders bevorzugt sind Cyclohexan, Methylcyclohexan und Toluol.

Im allgemeinen kann die Veresterung wie folgt durchgeführt werden:

Die Veresterungsapparatur besteht beispielsweise aus einem gerührten Reaktor, bevorzugt aus einem Reaktor mit Umlaufverdampfer und einer aufgesetzten Destillationseinheit.

Bei dem Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf, d.h. unter Verwendung einer Pumpe, besonders bevorzugt Naturumlauf, bei dem der Kreislaufstrom ohne mechanische Hilfsmittel bewerkstelligt wird, eingesetzt.

Selbstverständlich kann die Reaktion auch in mehreren Reaktionszonen, beispielsweise einer Reaktorkaskade aus zwei bis vier, bevorzugt zwei bis drei Reaktoren durchgeführt werden.

Geeignete Umlaufverdampfer sind dem Fachmann bekannt und beispielsweise beschrieben in R. Billet, "Verdampfertechnik", HTB-Verlag, Bibliographisches Institut Mannheim, 1965, Seite 53. Beispiele für Umlaufverdampfer sind Rohrbündelwärmetauscher, Plattenwärmetauscher, etc.

Selbstverständlich können im Umlauf auch mehrere Wärmetauscher vorhanden sein.

Um die partiell geschützten monoalkoxilierten Polyole zu hydrolysieren werden das partiell geschützte monoalkoxilierte Polyol, der Hydrolysekatalysator und Wasser in den Reaktor gegeben. Das Reaktionsgemisch wird mit Hilfe des Umlaufverdampfers zum Sieden erhitzt und die durch Hydrolyse abgespaltene Schutzgruppe, beispielsweise Aceton, gegebenenfalls abdestilliert.

Um die Veresterung durchzuführen, werden die Einsatzstoffe in den Reaktor gegeben. Das Reaktionsgemisch wird mit Hilfe des Umlaufverdampfers zum Sieden erhitzt und das bei der Veresterung gebildete Wasser wird als Azeotrop mit dem organischen Lösungsmittel abdestilliert. Dies erfolgt über eine auf den Reaktor aufgesetzte Destillationseinheit, die eine Destillationskolonne und einen Kondensator umfasst.

Vorzugsweise werden Hydrolyse und Veresterung nacheinander ohne Zwischenreinigung in einem Reaktor durchgeführt.

Die Destillationseinheit ist von an sich bekannter Bauart. Dabei kann es sich um eine einfache Destillation handeln, die gegebenenfalls mit einem Spritzschutz ausgestattet ist, oder um eine Rektifikationskolonne. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

In der Regel sind 5 bis 20 theoretische Böden ausreichend.

Der Kondensator ist ebenfalls von bekannter Bauart, beispielsweise kann es sich um Röhren- oder Plattenwärmetauscher handeln. Sie werden vorzugsweise mit Wasser oder Sole betrieben.

Die (Meth)acrylsäure und das monoalkoxilierte Polyol werden in der Veresterung in der Regel im molaren

Überschuss wie oben angegeben bezogen auf die Hydroxygruppen des Alkohols eingesetzt. Der eingesetzte Überschuss kann bis zu ca. 1000:1 betragen, falls gewünscht.

Als Veresterungskatalysatoren kommen die oben angeführten in Frage.

Das Polymerisationsinhibitor(gemisch) wird im allgemeinen in einer Gesamtmenge von 0,01 bis 1 Gew.-%, bezogen auf das Umesterungsgemisch, eingesetzt, bevorzugt 0,02 bis 0,8 Gew.-%, besonders bevorzugt 0,05 bis 0,5 Gew.-%.

Das Polymerisationsinhibitor(gemisch) kann beispielsweise als alkoholische Lösung oder als Lösung in einem Edukt oder Produkt eingesetzt werden.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich einer Kolonne und/oder in einen Umlaufverdampfer eindosiert und/oder durch das Reaktionsgemisch und/oder über dieses geleitet.

Das bei der Reaktion entstehende Wasser kann während oder nach der Veresterung abdestilliert werden, wobei dieser Vorgang durch ein mit Wasser ein Azeotrop bildendes Lösungsmittel unterstützt werden kann.

Als Lösungsmittel zur azeotropen Entfernung des Wassers, falls gewünscht, eignen sich die oben angeführten Kohlenwasserstoffe.

Bevorzugt ist die Durchführung der Veresterung in Gegenwart eines Überschusses an (Meth)acrylsäure.

Falls das aus dem Reaktionsgemisch zu entfernende Wasser nicht über ein azeotropbildendes Lösungsmittel entfernt wird, so ist es möglich, dieses über Strippen mit einem inerten Gas, bevorzugt einem sauerstoffhaltigen Gas, besonders bevorzugt mit Luft oder Magerluft zu entfernen.

Die Reaktionstemperatur der Veresterung liegt allgemein bei 40 bis 160°C, bevorzugt bei 60 bis 140°C, und besonders bevorzugt bei 80 bis 120°C. Die Temperatur kann im Reaktionsverlauf gleichbleiben oder ansteigen, bevorzugt wird sie im Reaktionsverlauf angehoben. In diesem Fall ist die Endtemperatur der Umesterung um 5 bis 30°C höher als die Anfangstemperatur. Die Temperatur der Veresterung kann durch Variation der Lösungsmittelkonzentration im Reaktionsgemisch oder durch Variation des Reaktionsdruckes geregelt werden.

Bevorzugt wird das Azeotrop aus dem gebildeten Wasser und dem organischen Lösungsmittel über die Destillationskolonne abgetrennt und anschließend im Kondensator kondensiert, wobei das Kondensat in eine Wasserphase und eine organische Phase zerfällt. Die Wasserphase wird zumindest teilweise ausgeschleust oder kann zur Gewinnung der darin enthaltenen (Meth)acrylsäure der weiteren Verarbeitung zugeführt werden. Die organische Phase stellt den Rücklauf dar und dieser wird zumindest teilweise in den Kreislauf zwischen Reaktor und Umlaufverdampfer geleitet. Vorzugsweise werden mindestens 10 Gew.-% des Rücklaufs in den Kreislauf geleitet. Dabei kann der Rücklauf in die vom Reaktor zum Umlaufverdampfer führende und den Zulauf zum Umlaufverdampfer bildende Leitung oder alternativ in den Umlaufverdampfer im Bereich des Zulaufs eingespeist werden. Nach Durchlaufen des Umlaufverdampfers wird das Reaktionsgemisch in den Reaktor zurückgeführt.

Der Rücklauf kann, wie in der Patentanmeldung DE-A-199 41 136 beschrieben, zur Steuerung der Temperatur in der Umesterung verwendet werden.

Die Veresterung kann drucklos aber auch bei Überdruck oder Unterdruck durchgeführt werden, vorzugsweise wird bei Normaldruck oder Unterdruck, besonders bevorzugt bei einem Reaktionsdruck von 200 bis 1013 mbar, gearbeitet.

Die Reaktionszeit beträgt in der Regel 2 bis 20 Stunden, vorzugsweise 4 bis 15 und besonders bevorzugt 7 bis 12 Stunden.

Die Reihenfolge der Zugabe der einzelnen Reaktionskomponenten ist nicht wesentlich. Es können alle Komponenten gemischt vorgelegt und anschließend aufgeheizt werden oder es können eine oder mehrere Komponenten nicht oder nur teilweise vorgelegt und erst nach dem Aufheizen zugegeben werden.

Wird die Veresterung in einem Reaktor mit Naturumtaufverdampfer durchgeführt, so ist es zweckmäßig die niedriger siedenden Reaktionskomponenten zumindest teilweise vorzulegen.

Die einsetzbare (Meth)acrylsäure ist in ihrer Zusammensetzung nicht beschränkt und kann beispielsweise folgende Komponenten aufweisen:

| | |
|---|---|
| (Meth)acrylsäure | 90 - 99,9 Gew% |
| Essigsäure | 0,05 - 3 Gew% |
| Propionsäure | 0,01 - 1 Gew% |
| Diacrylsäure | 0,01 - 5 Gew% |
| Wasser | 0,05 - 5 Gew% |
| Carbonylhaltige | 0,01 - 0,3 Gew% |
| Inhibitoren | 0,01 - 0,1 Gew% |
| Maleinsäure(-anhydrid) | 0,001 - 0,5 Gew% |

Die eingesetzte Roh-(Meth)acrylsäure ist in der Regel stabilisiert mit 200 - 600 ppm Phenothiazin oder anderen Stabilisatoren in Mengen, die eine vergleichbare Stabilisierung ermöglichen. Unter dem Ausdruck Carbonylhaltige werden hier beispielsweise Aceton und niedere Aldehyde, wie z.B. Formaldehyd, Acetaldehyd, Crotonaldehyd, Acrolein, 2- und 3- Furfural und Benzaldehyd, verstanden.

Unter Roh-(Meth)acrylsäure wird hier das (meth)acrylsäurehaltige Gemisch verstanden, das nach Absorption der Reaktionsgase der Propan/Propen/Acrolein- beziehungsweise Isobutan/Isobuten/Methacrolein-Oxidation in einem Absorptionsmittel und anschließender Abtrennung des Absorptionsmittels anfällt beziehungsweise das durch fraktionierende Kondensation der Reaktionsgase gewonnen wird.

Selbstverständlich kann auch Rein-(Meth)acrylsäure eingesetzt werden, die im wesentlichen frei von den aldehydischen, anderen carbonylhaltigen und hochsiedenden Komponenten ist, mit beispielsweise einer Reinheit von größer 99,5 Gew.-%.

Die während der Veresterung abdestillierte wässrige Phase des über die aufgesetzte Kolonne, wenn vorhanden, abgetrennten Kondensats, die in der Regel 0,1 - 10 Gew% (Meth)acrylsäure enthalten kann, wird abgetrennt und ausgeschleust. Vorteilhaft kann die darin enthaltene (Meth)acrylsäure mit einem Extraktionsmittel, bevorzugt dem gegebenenfalls in der Veresterung verwendeten Lösungsmittel, beispielsweise mit Cyclohexan, bei einer Temperatur zwischen 10 und 40 °C und einem Verhältnis von wässriger Phase zu Extraktionsmittel von 1 : 5 - 30, bevorzugt 1 : 10 - 20, extrahiert und in die Veresterung zurückgeführt werden.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) von unten in den Umlaufverdampfer geleitet werden, beispielsweise in Mengen von 0,1 bis 1 $m^3/m^3h$, bevorzugt 0,2 bis 0,8 $m^3/m^3h$, und besonders bevorzugt 0,3 bis 0,7 $m^3/m^3h$, bezogen auf das Volumen des Reaktionsgemisches.

Der Verlauf der Veresterung kann durch Verfolgung der ausgetragenen Wassermenge verfolgt werden.

Die Reaktion kann beispielsweise beendet werden, sobald 90 % der theoretisch zu erwartenden Wassermenge ausgetragen worden sind, bevorzugt bei mindestens 95% und besonders bevorzugt bei mindestens 98%.

Nach Beendigung der Veresterung wird der saure Katalysator auf übliche Weise zerstört oder entfernt. Dies geschieht bei Homogenkatalysatoren, wie beispielsweise Schwefelsäure, durch Neutralisation, vorzugsweise mit Natronlauge; und bei Heterogenkatalysatoren, wie beispielsweise sauren Ionenaustauschern, durch Filtration.

In einer weiteren Ausführungsform kann die Reaktionsmischung nach beendeter Umesterung mit Wasser verdünnt werden und auf eine Konzentration von beispielsweise 10 bis 90 Gew.-%, bevorzugt 20 bis 80 Gew.-%, besonders bevorzugt 20 bis 60 Gew.-%, ganz besonders bevorzugt 30 bis 50 Gew.-%, und insbesondere ca. 40 Gew.-%, verdünnt werden, beispielsweise um die Viskosität zu verringern.

Falls erforderlich kann das Reaktionsgemisch einer Entfärbung, beispielsweise durch Behandlung mit Aktivkohle oder Metalloxiden, wie beispielsweise Aluminiumoxid, Siliciumoxid, Magnesiumoxid, Zirkonoxid, Boroxid oder Gemischen davon, in Mengen von beispielsweise 0,1 bis 50 Gew.-%, bevorzugt 0,5 bis 25 Gew.-%, besonders bevorzugt 1 bis 10 Gew.-%, bei Temperaturen von beispielsweise 10 bis 100°C, bevorzugt 20 bis 80°C, und besonders bevorzugt 30 bis 60°C, unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Die Entfärbung des Reaktionsgemisches kann an beliebiger Stelle des Aufarbeitungsverfahrens erfolgen, beispielsweise auf der Stufe des rohen Reaktionsgemisches oder nach gegebenenfalls erfolgter Neutralisation oder nach Entfernung des Lösungsmittels.

Das gegebenenfals für die Veresterung eingesetzte und im Reaktionsgemisch enthaltene Lösungsmittel kann durch Destillation im wesentlichen entfernt werden. Dabei werden auch im Reaktionsgemisch enthaltene Leichtsieder entfernt. Leichtsieder sind Komponenten mit einem Siedepunkt unterhalb des Siedepunkts des Zielesters.

Die destillative Abtrennung der Hauptmenge des Lösungsmittels erfolgt beispielsweise in einem Rührkessel mit Doppelwandheizung und/oder innenliegenden Heizschlangen unter vermindertem Druck, beispielsweise bei 20 bis 700 mbar, bevorzugt 30 bis 500 mbar, und besonders bevorzugt 50 bis 150 mbar, und einer Temperatur von 40 bis 120°C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 bis 700 mbar, bevorzugt 30 bis 500 mbar, und besonders bevorzugt 50 bis 150 mbar, und einer Temperatur von 40 bis 120°C durch den Apparat geführt.

Bei der Verwendung von Wasser als Verdünnungsmittel kann das gegebenenfalls enthaltene Lösungsmittel azeotrop abdestilliert werden. Dabei kann das Destillat, nach Kondensation, in einen Phasentrennapparat geführt werden. Die so erhaltene organische Phase kann ausgeschleust werden, die wässrige Phase kann ebenfalls ausgeschleust oder als Rücklauf in die Destillationseinheit geführt werden.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 bis 1

$m^3/m^3h$, bevorzugt 0,2 bis 0,8 $m^3/m^3h$, und besonders bevorzugt 0,3 bis 0,7 $m^3/m^3h$, bezogen auf das Volumen des Reaktionsgemisches.

Der Gehalt an (Meth)acrylsäure im Rückstand beträgt nach der Destillation in der Regel unter 5 Gew.-%, bevorzugt 0,001 bis 5 Gew.-%, und besonders bevorzugt 0,01 bis 3 Gew.-%.

Das abgetrennte Lösungsmittel kann kondensiert und bevorzugt wiederverwendet werden.

Falls erforderlich kann zusätzlich oder anstelle der Destillation eine Lösungsmittelstrippung durchgeführt werden.

Dazu wird der Zielester, der noch geringe Mengen an (Meth)acrylsäure und/oder organisches Lösungsmittel enthält, auf 50 bis 90°C, bevorzugt 80 bis 90°C, erwärmt und die restlichen Leichtsieder mit einem geeigneten Gas in einer geeigneten Apparatur entfernt. Zur Unterstützung kann gegebenenfalls auch Vakuum angelegt werden.

Geeignete Apparaturen sind beispielsweise Kolonnen von an sich bekannter Bauart, die die üblichen Einbauten, beispielsweise Böden, Schüttungen oder gerichtete Packungen, bevorzugt Schüttungen aufweisen. Als Kolonneneinbauten kommen prinzipiell alle gängigen Einbauten in Betracht, beispielsweise Böden, Packungen und/oder Füllkörper. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten, bevorzugt.

Denkbar ist hier auch ein Fallfilm-, Dünnfilm- oder Wischfilmverdampfer, wie beispielsweise ein Luwa-, Rotafilm- oder Sambayverdampfer, der als Spritzschutz beispielsweise mit einem Demister ausgerüstet sein kann.

Geeignete Gase sind unter den Strippbedingungen inerte Gase, bevorzugt sauerstoffhaltige Gase, besonders bevorzugt Luft oder Gemische aus Luft und Stickstoff (Magerluft) oder Wasserdampf, insbesondere solche, die auf 50 bis 100°C temperiert sind.

Die Strippgasmenge beträgt beispielsweise 5 bis 20 $m^3/m^3h$, besonders bevorzugt 10 bis 20 $m^3/m^3h$, und ganz besonders bevorzugt 10 bis 15 $m^3/m^3h$, bezogen auf das Volumen des Reaktionsgemisches.

Falls notwendig kann der (Meth)acrylsäureester des monoalkoxilierten Polyols in einem beliebigen Stadium des Aufarbeitungsverfahrens, bevorzugt nach erfolgter Leichtsiederentfernung, einer Filtration unterworfen werden, um ausgefallene Spuren an Salzen sowie gegebenenfalls enthaltenes Entfärbungsmittel zu entfernen.

Die nach dem obigen Verfahren erhältlichen (Meth)acrylsäureester monoalkoxilierter Polyole und erfindungsgemäßen wässrigen Lösungen können Verwendung finden
als Radikalvernetzer von wasserabsorbierenden Hydrogelen,
als Ausgangsstoff für die Herstellung von Polymerdispersionen,
als Ausgangsstoff für die Herstellung von Polyacrylaten (außer Hydrogelen),
als Lackrohstoff oder
als Zementadditiv.

Zur Verwendung als Radikalvernetzer von wasserabsorbierenden Hydrogelen sind insbesondere solche erfindungsgemäßen (Meth)acrylsäureester monoalkoxilierter Polyole geeignet, die eine Wasserlöslichkeit (bei 25°C in destilliertem Wasser) von mindestens 5 Gew.-%, bevorzugt mindestens 10 Gew.-%, besonders bevorzugt mindestens 20 Gew.-%, ganz besonders bevorzugt mindestens 30 Gew.-%, und insbesondere von mindestens 50 Gew.-% aufweisen.

Zur Herstellung der vernetzten quellbaren hydrogelbildenen Polymere geeignete hydrophile Monomere sind beispielsweise polymerisationsfähige Säuren, wie Acrylsäure, Methacrylsäure, Ethacrylsäure, α-Chloracrylsäure, Crotonsäure, Maleinsäure, Maleinsäureanhydrid, Vinylsulfonsäure, Vinylphosphonsäure, Maleinsäure einschließlich deren Anhydrid, Fumarsäure, Itaconsäure, Citraconsäure, Mesaconsäure, Glutaconsäure, Aconitsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Allylphosphonsäure, Styrolsulfonsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide. Die Monomeren können allein oder in Mischung untereinander eingesetzt werden. Des weiteren wasserlösliche N-Vinylamide oder auch Diallyldimethylammoniumchlorid.

Besonders bevorzugte hydrophile Monomere sind Acrylsäure und Methacrylsäure.

Zur Optimierung von Eigenschaften kann es sinnvoll sein, zusätzliche monoethylenisch ungesättigte Verbindungen einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäuren, wie beispielsweise Acrylamid, Methacrylamid und N-Vinylformamid, N-Vinylacetamid, N-Methyl-vinylacetamid, Acrylnitril und Methacrylnitril. Weitere geeignete Verbindungen sind beispielsweise Vinylester von gesättigten $C_1$- bis $C_4$-Carbonsäuren wie Vinylformiat, Vinylacetat oder Vinylpropionat, Alkylvinylether mit mindestens zwei C-Atomen in der Alkylgruppe, wie beispielsweise Ethylvinylether oder Butylvinylether, Ester von monoethylenisch ungesättigten $C_3$- bis $C_6$-Carbonsäuren, wie beispielsweise Ester aus einwertigen $C_1$- bis $C_{18}$-Alkoholen und Acrylsäure, Methacrylsäure oder Maleinsäure, Halbester von Maleinsäure, wie beispielsweise Maleinsäure-monomethylester, N-Vinyllactame wie N-Vinylpyrrolidon oder N-Vinylcaprolactam, Acrylsäure- und Methacrylsäureester von alkoxilierten einwertigen, gesättigten Alkoholen, wie beispielsweise von Alkoholen mit 10 bis 25 C-Atomen, die mit 2 bis 200 Mol Ethylenoxid und/oder

Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, wie beispielsweise Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-%, zugesetzt.

Bevorzugt bestehen die vernetzten (Co)Polymere aus Säuregruppen tragenden monoethylenisch ungesättigten Monomeren, die gegebenenfalls vor oder nach der Polymerisation in ihre Alkali- oder Ammoniumsalze überführt werden, und aus 0 bis 40

Gew.-% bezogen auf ihr Gesamtgewicht keine säuregruppentragenden monoethylenisch ungesättigten Monomeren.

Die Herstellung (meth)acrylsäurehaltiger (Co)Polymere, Polyacrylsäuren und Superabsorbern ist vielfach beschrieben, siehe beispielsweise "Modern Superabsorbent Polymer Technology", F.L. Buchholz and A.T. Graham, Wiley-VCH, 1998, Seiten 69 bis 117.

Bevorzugt sind solche Hydrogele, die durch vernetzende Polymerisation oder Copolymerisation von säuregruppentragenden monoethylenisch ungesättigten Monomeren oder deren Salzen erhalten werden.

Bei dem Verfahren zur Nachvernetzung wird das Ausgangspolymer mit einem Nachvernetzer behandelt und bevorzugt während oder nach dem Behandeln durch Temperaturerhöhung nachvernetzt und getrocknet, wobei der Vernetzer bevorzugt in einem inerten Lösemittel enthalten ist. Unter inerten Lösemittel werden solche verstanden, die bei der Reaktion weder mit dem Ausgangspolymer noch mit dem Nachvernetzer im wesentlichen reagieren. Bevorzugt sind solche Lösemittel, die zu mehr als 90%, bevorzugt mehr als 95%, besonders bevorzugt mehr als 99%, insbesondere zu mehr als 99,5%, nicht chemisch mit dem Ausgangspolymer oder Nachvernetzer reagieren.

Bevorzugt zur Nachvernetzung und Trocknung ist dabei der Temperaturbereich zwischen 30 und 250°C, insbesondere 50 und 200°C, ganz besonders bevorzugt ist der Bereich zwischen 100 und 180°C. Die Aufbringung der Oberflächennachvemetzungslösung erfolgt bevorzugt durch Aufsprühen auf das Polymere in geeigneten Sprühmischern. Im Anschluss an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischem oder Misch- und Trocknungsanlagen wie beispielsweise Lödige-Mischer, BEPEX-Mischer, NAUTA-Mischer, SHUGGI-Mischer oder PROCESSALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie beispielsweise ein Hordentrockner, ein Drehrohrofen, oder eine beheizbare Schnecke. Es kann aber auch beispielsweise eine azeotrope Destillation als Trocknungsverfahren benutzt werden. Die bevorzugte Verweilzeit bei dieser Temperatur im Reaktionsmischer oder Trockner beträgt unter 60 Minuten, besonders bevorzugt unter 30 Minuten.

Bevorzugt sind obige Verfahren, wobei das Ausgangspolymere eine polymere (Meth)acrylsäure oder ein Poly (meth)acrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

Bevorzugt werden solche Verfahren in denen der Radikalvernetzer in einer Dosierung von 0,01 bis 5,0 Gew.-%, bevorzugt 0,02 bis 3,0 Gew.-%, ganz besonders bevorzugt 0,03 bis 2,5 Gew.-%, insbesondere 0,05 bis 1,0 Gew.-%, und speziell 0,1 bis 0,75 Gew.-%, bezogen auf das Ausgangspolymer verwendet wird.

Gegenstand der Erfindung sind auch Polymere, hergestellt nach einem der oben genannten Verfahren und deren Verwendung in Hygieneartikeln, Verpackungsmaterialien und in Nonwovens, sowie die Verwendung von einem oben genannten Stoffgemisch zur Herstellung von vernetzten oder durch Wärmebehandlung vernetzungsfähigen Polymeren, insbesondere in Lacken und Farben.

Die dabei einzusetzenden vernetzten quellbaren hydrogelbildenen Polymere (Ausgangspolymere) sind insbesondere Polymere aus (co)polymerisierten hydrophilen Monomeren, Pfropf(co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Diese Hydrogele sind dem Fachmann bekannt und beispielsweise beschrieben in US-4,28, 082, DE-C-27 06 135, US-4,340,706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-4219 607, DE-A-40 21 847, DE-A-38 31 261, DE-A-35 11 086, DE-A-31 18 172, DE-A-30 28 043, DE-A-4418 881, EP-A-0 801 483, EP-A-0 455 985, EP-A-0 467 073, EP-A-0 312 952, EP-A-0 205 874, EP-A-0 499 774, DE-A-26 12 846, DE-A-40 20 780 EP-A-0 205 674, US-5,145,906, EP-A-0 530 438, EP-A-0 670 073, US-4,057,521, US-4,062,817, US-4,525,527, US-4,295,987, US-5,011,892, US-4,076,663 oder US-4,931,497. Besonders geeignet sind auch vernetzte quellbare hydrogelbildene Polymere aus einem Herstellprozess wie in der Patentanmldung WO 01/38402 beschrieben, sowie anorganisch-organische hybride vernetzte quellbare hydrogelbildene Polymere wie

in der Patentanmeldung DE-A-198 54 575 beschrieben. Der Inhalt der vorstehend genannten Patentdokumente, insbesondere die nach den Verfahren hergestellten Hydrogele, sind ausdrücklich Bestandteil der vorliegenden Offenbarung.

Geeignete Pfropfgrundlagen für vernetzte quellbare hydrogelbildene Polymere, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Das vernetzte quellbare hydrogelbildene Polymer kann über radikalische Pfropfcopolymerisation von Acrylsäure oder Acrylat auf eine wasserlösliche Polymermatrix erhalten werden. Geeignete wasserlösliche Polymermatrices sind beispielsweise, aber nicht ausschliesslich, Alginate, Polyvinylalkohol, und Polysacharide wie etwa Stärke. Bei der Pfropfcopolymerisation im erfindungsgemäßen Sinne wird ein mehrfunktioneller ethylenisch ungesättigter Radikalvernetzer eingesetzt.

Das vernetzte quellbare hydrogelbildene Polymer kann ein organisch-anorganisches Hybridpolymer aus einer polymeren Acrylsäure oder einem Polyacrylat einerseits und einem Silikat, Aluminat, oder Alumosilikat andererseits sein. Insbesondere können polymere Acrylsäure oder Polyacrylat verwendet werden, die über radikalische Polymerisation erhalten wurden, und bei denen ein mehrfunktioneller ethylenisch ungesättigter Radikalvernetzer verwendet wurde und bei deren Herstellprozess ein in Wasser lösliches Silikat oder lösliches Aluminat oder Gemische von beiden eingesetzt wurde.

Bevorzugte vernetzte quellbare hydrogelbildene Polymere sind insbesondere Polyacrylate, Polymethacrylate sowie die in US-4,931,497, US-5,011,892 und US-5,041,496 beschriebene Pfropfpolymere. Ganz besonders bevorzugte vernetzte quellbare hydrogelbildene Polymere sind die in WO 01/38402 beschriebenen Kneterpolymere und die in DE-A-198 545 75 beschriebenen hybriden organisch-anorganischen vernetzten quellbaren hydrogelbildenden Polymere auf Basis von Polyacrylaten.

Die erfindungsgemäß hergestellten, als Radikalvernetzer in vernetzten quellbaren hydrogelbildenen Polymeren verwendbaren Substanzen können allein oder in Kombination mit anderen Vernetzern, beispielsweise Innen- oder Oberflächenvemetzern verwendet werden, beispielsweise den folgenden:

Methylenbisacryl- bzw. -methacrylamid, Ester ungesättigter Mono- oder Polycarbonsäuren von Polyolen, wie Diacrylat oder Triacrylat, z. B. Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weitere geeignete Co-Vemetzer sind Pentaerythrittri- und -tetraallylether, Polyethylenglykoldiallylether, Monoethylenglykol-diallylether, Glyceroldi- und Triallylether, Polyallylether auf Basis Sorbitol, sowie ethoxilierte Varianten davon. Weiterhin besonders bevorzugte Co-Vernetzer sind die Polyethylenglykoldiacrylate, ethoxilierte Derivate von Trimethylolpropantriacrylat wie beispielsweise Sartomer® SR 9035 der Firma Sartomer, sowie ethoxilierte Derivate von Glycerindiacrylat und Glycerintriacrylat. Selbstverständlich können auch Gemische der obigen Vernetzer eingesetzt werden.

Ganz besonders bevorzugt sind solche vernetzten quellbaren hydrogelbildenden Polymere, die mit einem erfindungsgemäß hergestellten (Meth)acrylsäureester eines monoalkoxilierten Polyols als einzigen Radikalvernetzer hergestellt werden.

Das vernetzte quellbare hydrogelbildene Polymer ist bevorzugt eine polymere Acrylsäure oder ein Polyacrylat

Die vernetzten quellbaren hydrogelbildenen Polymere können durch an sich bekannte Polymerisationsverfahren hergestellt werden. Bevorzugt ist die Polymerisation in wässriger Lösung nach dem Verfahren der sogenannten Gelpolymerisation. Dabei werden wie oben angeführt verdünnte, bevorzugt wässrige, besonders bevorzugt 15 bis 50 gew.-%ige wässrige Lösungen eines oder mehrerer hydrophiler Monomerer und gegebenenfalls einer geeigneten Pfropfgrundlage in Gegenwart eines Radikalinitiators bevorzugt ohne mechanische Durchmischung unter Ausnutzung des Trommsdorff-Norrish-Effektes (Makromolekulare Chemie, Band 1, Jahrgang 1947, Seiten 169 ff), polymerisiert. Die Polymerisationsreaktion kann im Temperaturbereich von 0 bis 150°C, vorzugsweise von 10 bis 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$.

Sie können gegebenenfalls in Kombination mit Reduktionsmitteln wie beispielsweise Ascorbinsäure, Natriumhydrogensulfit, und Eisen(II)- sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie beispielsweise Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich von 50 bis 130°C, vorzugsweise von 70 bis 100°C, können die Qualitätseigenschaften der Polymere noch verbessert werden.

Die erhaltenen Gele werden zu 0 bis 100 mol-%, bevorzugt zu 25 bis 100 mol-%, und besonders bevorzugt zu 50 bis 85 mol-% bezogen auf eingesetztes Monomer neutralisiert, wobei die üblichen Neutralisationsmittel verwendet werden können, bevorzugt Alkalimetallhydroxide, Alkalimetalloxide oder die entsprechenden Alkalimetallcarbonate, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, beispielsweise mittels eines Fleischwolfes, und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt.

Die Polymerisation an sich kann aber auch nach jedem anderen der in der Literatur beschriebenen Verfahren durchgeführt werden. Insbesondere kann die Neutralisation der Acrylsäure auch vor der Polymerisation durchgeführt werden, wie oben beschrieben. Die Polymerisation kann dann in einem dem Fachmann bekannten Bandreaktor oder einem Knetreaktor kontinuierlich oder auch diskontinuierlich durchgeführt werden. Bei Durchführung der Polymerisation in einem Bandreaktor ist die Initiation mittels elektromagnetischer Strahlung, bevorzugt mittels UV-Strahlung, oder alternativ die Initiation mit einem Redoxinitiatorsystem besonders bevorzugt. Ganz besonders bevorzugt ist auch die Kombination beider Initiationsmethoden: elektromagnetische Strahlung und chemisches Redoxinitiatorsystem simultan.

Das getrocknete vernetzte quellbare hydrogelbildene Polymer kann hiemach gemahlen und gesiebt werden, wobei zur Mahlung üblicherweise Walzenstühle, Stiftmühlen oder Schwingmühlen eingesetzt werden können. Die bevorzugte Partikelgröße des gesiebten Hydrogels liegt vorzugsweise im Bereich 45 bis 1000 μm, bevorzugt bei 45 bis 850 μm, besonders bevorzugt bei 200 bis 850 μm, und ganz besonders bevorzugt bei 300 bis 850 μm. In diesen Bereichen liegen bevorzugt 80 Gew.-% der Teilchen, insbesondere 90 Gew.-% der Teilchen. Die Größenverteilung kann mit etablierten Lasermethoden bestimmt werden.

Gegenstand der vorliegenden Erfindung sind weiterhin vernetzte quellbare hydrogelbildende Polymere, die mindestens ein hydrophiles Monomer in einpolymerisierter Form enthalten und vemetzt sind mit einem (Meth)acrylsäureeester eines monoalkoxilierten Polyols der Formel (I).

Bevorzugte (Meth)acrylsäureeester monoalkoxilierter Polyole sind solche der Formel (I), wie oben definiert.

Die durch die Formel (II) beschriebenen monoalkoxilierten Polyole, deren (Meth)acrylsäureester als Vernetzer in den vorstehend genannten vernetzten quellbaren hydrogelbildenden Polymeren verwendet werden, sind jeweils alkoxiliert, vorzugsweise ethoxiliert, propoxiliert oder gemischt ethoxiliert und propoxiliert und insbesondere ethoxiliert oder gemischt ethoxiliert und propoxiliert und ganz besonders bevorzugt ausschließlich ethoxiliert.

Besonders bevorzugte (Meth)acrylsäureeester monoalkoxilierter Polyole sind solche der Formel (I), deren monoalkoxilierte Polyole sich von Isopropylidenglycerin und Isopropylidentrimethylolpropan ableiten.

Der CRC-Wert [g/g] der erfindungsgemäßen vernetzten quellbaren hydrogelbildenden Polymere kann nach den in der Beschreibung angegebenen Methoden gemessen werden und ist bevorzugt größer 15, insbesondere größer 20, besonders bevorzugt größer 25, insbesondere größer 30, insbesondere bevorzugt größer 35.

Der AUL-0,7psi-Wert [g/g] der erfindungsgemäßen vernetzten quellbaren hydrogelbildenden Polymere kann nach den in der Beschreibung angegebenen Methoden gemessen werden und ist nach der Nachvernetzung bevorzugt größer 5, insbesondere größer 10, besonders bevorzugt größer 15, insbesondere größer 20, insbesondere bevorzugt größer 25.

Die vorliegende Erfindung betrifft ferner die Verwendung der oben genannten vernetzten quellbaren hydrogelbildenden Polymere in Hygieneartikeln. Beispielsweise kann der Hygieneartikel wie folgt aufgebaut sein:

(A) eine obere flüssigkeitsdurchlässige Abdeckung
(B) eine untere flüssigkeitsundurchlässige Schicht
(C) einen zwischen (A) und (B) befindlichen Kern, enthaltend
10 bis 100 Gew.-% des erfindungsgemäßem vernetzten quellbaren hydrogelbildenden Polymers
0 bis 90 Gew.-% hydrophiles Fasermaterial
bevorzugt 30 bis 100 Gew.-% des erfindungsgemäßem vernetzten quellbaren hydrogelbildenden Polymers, 0 bis 70 Gew.-% hydrophiles Fasermaterial
besonders bevorzugt 50 bis 100 Gew.-% des erfindungsgemäßem vernetzten quellbaren hydrogelbildenden Polymers 0 bis 50 Gew.-% hydrophiles Fasermaterial insbesondere bevorzugt 70 bis 100 Gew.-% des erfindungsgemäßem vernetzten quellbaren hydrogelbildenden Polymers, 0 bis 30 Gew.-% hydrophiles Fasermaterial am meisten bevorzugt 90 bis 100 Gew.-% des erfindungsgemäßem vernetzten quellbaren hydrogelbildenden Polymers, 0 bis 10 Gew.-% hydrophiles Fasermaterial
(D) gegebenenfalls eine sich unmittelbar oberhalb und unterhalb des Kerns (C) sich befindende Tissueschicht und
(E) gegebenenfalls eine zwischen (A) und (C) sich befindende Aufnahmeschicht.

Unter Hygieneartikel sind dabei beispielsweise Inkontinenzeinlagen und Inkontinenzhosen für Erwachsene oder Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355, EP-A-1 023 883.

Die flüssigkeitsundurchlässige Schicht (B) besteht in der Regel aus einer Folie aus Polyethylen oder Polypropylen.

Der Kern (C) enthält neben dem erfindungsgemäßen vernetzten quellbaren hydrogelbildenden Polymer hydrophiles Fasermaterial. Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 bis 200 µm, bevorzugt 10 bis 100 µm. Darüberhinaus haben die Fasern eine Mindestlänge von 1 mm.

Der Aufbau und die Form von Windeln ist allgemein bekannt und beispielsweise in der WO 95/26209 Seite 66, Zeile 34 bis Seite 69, Zeile 11, DE-A-196 04 601, EP-A-0 316 518 und EP-A-0 202 127 beschrieben. Allgemein werden Windeln und andere Hygieneartikel auch in WO 00/65084, insbesondere auf Seiten 6 bis 15, WO 00/65348, insbesondere auf Seiten 4 bis 17, WO 00/35502, insbesondere Seiten 3 bis 9, DE-A 197 37 434 und WO 98/08439 beschrieben. Hygieneartikel für die Damenhygiene werden in folgenden Literaturstellen beschrieben. Die erfindungsgemäßen wässrige Flüssigkeiten absorbierende vernetzten quellbaren hydrogelbildenden Polymere können dort eingestzt werden. Literaturstellen Damenhygiene: WO 95/24173: Absorption Article for Controlling Odour, WO 91/11977: Body Fluid Odour Control, EP-A-0 389 023: Absorbent Sanitary Articles, WO 94/25077: Odour Control Material, WO 97/01317: Absorbent Hygienic Article, WO 99/18905, EP-A-0 834 297, US-5,762,644; US-5,895,381, WO 98/57609, WO 00/65083, WO 00/69485, WO 00/69484, WO 00/69481, US-6,123,693, EP-A-1 104 666, WO 01/24755, WO 01/00115, EP-A-0 105 373, WO 01/41692, EP-A-1 074 233. Tampons werden in folgenden Schriften beschrieben: WO 98/48753, WO 98/41179, WO 97/09022, WO 98/46182, WO 98/46181, WO 01/43679, WO 01/43680, WO 00/61052, EP-A-1 108 408, WO 01/33962, DE-A-100 20 662, WO 01/01910, WO 01/01908, WO 01/01909, WO 01/01906, WO 01/01905, WO 01/24729. Inkontinenzartikel werden in folgenden Schriften beschrieben: Disposable Absorbent Article for Incontinent Individuals: EP-A-0 311 344 Beschreibung Seiten 3 bis 9, Disposable Absorbent Article: EP-A-0 850 623, Absorbent Article: WO 95/26207, Absorbent Article: EP-A-0 894 502, Dry Laid Fibrous Structure: EP-A-0 850 616, WO 98/22063, WO 97/49365, EP-A-0 903 134, EP-A-0 887 060, EP-A-0 887 059, EP-A-0 887 058, EP-A-0 887 057, EP-A-0 887 056, EP-A-0 931 530, WO 99/25284, WO 98/48753. Damenhygiene- und Inkontinenzartikel wird in folgenden Schriften beschrieben: Catamenial Device: WO 93/22998 Beschreibung Seiten 26 bis 33, Absorbent Members for Body Fluids: WO 95/26209 Beschreibung Seiten 36 bis 69, Disposable Absorbent Article: WO 98/20916 Beschreibung Seiten 13 bis 24, Improved Composite Absorbent Structures: EP-A-0 306 262 Beschreibung Seiten 3 bis 14, Body Waste Absorbent Article: WO 99/45973. Diese Referenzen werden hiermit ausdrücklich in die Offenbarung der Erfindung einbezogen.

Die erfindungsgemäßen vernetzten quellbaren hydrogelbildenden Polymere eignen sich hervorragend als Absorptionsmittel für Wasser und wässrige Flüssigkeiten, so dass sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Zusätzlich zu den oben beschriebenen vernetzten quellbaren hydrogelbildenden Polymeren liegen in der absorbierenden Zusammensetzung gemäß der vorliegenden Erfindung Kompositionen vor, welche die vernetzten quellbaren hydrogelbildenden Polymere enthalten oder an denen sie fixiert sind. Jede Komposition ist geeignet, die die vernetzte quellbare hydrogelbildende Polymere aufnehmen kann und die darüber hinaus in die Absorptionsschicht integriert werden kann. Eine Vielzahl derartiger Zusammensetzungen ist bereits bekannt. Eine Komposition zum Einbau der vernetzten quellbaren hydrogelbildenden Polymere kann beispielsweise eine Fasermatrix sein, die aus einem Cellulosefasergemisch (airlaid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozess eines airlaid web ist beispielsweise geschildert in der Patentanmeldung WO 98/28478. Des weiteren können offenporige Schäume oder ähnliches zum Einbau vernetzter quellbarer hydrogelbildender Polymere dienen.

Alternativ kann eine derartige Komposition durch Fusion zweier Einzelschichten entstehen, wobei eine oder besser eine Vielzahl an Kammern gebildet werden, die die vernetzten quellbaren hydrogelbildenden Polymere enthalten. Ein derartiges Kammersystem ist detailliert geschildert in der Patentanmeldung EP-A-0 615 736 Seite 7, Zeilen 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum

Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die vernetzten quellbaren hydrogelbildenden Polymerpartikel zurückzuhalten. Obige Beispiele zur Komposition der absorbierenden Zusammensetzung schließen auch Laminate aus mindestens zwei Schichten mit ein, zwischen die die vernetzten quellbaren hydrogelbildenden Polymere eingebaut und fixiert werden.

Generell besteht die Möglichkeit, Hydrogelpartikel innerhalb des Absorbent Cores zur Verbesserung der sogenannte Dry- und Wet-Integrity zu fixieren. Unter Dry- und Wet-Integrity versteht man die Fähigkeit, vernetzte quellbare hydrogelbildende Polymere derart in die absorbierende Zusammensetzung einzubauen, dass sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in der Patentanmeldung WO 95/26209, Seite 37, Zeile 36 bis Seite 41, Zeile 14. Besagte Passage ist somit Bestandteil dieser Erfindung. Methoden zur Erhöhung der Wet Strength finden sich auch in der Patentanmeldung WO 00/36216.

Des weiteren kann die absorbierende Zusammensetzung aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die vernetzten quellbaren hydrogelbildenden Polymere mit einem Gewichtsanteil von 10 bis 100 Gew.-%, bevorzugt 30 bis 100 Gew.-%, besonders bevorzugt 50 bis 100 Gew.-%, insbesondere bevorzugt 70 bis 100 Gew.-%, und am meisten bevorzugt 90 bis 100 Gew.-%, basierend auf dem Gesamtgewicht der Komposition und der vernetzten quellbaren hydrogelbildenden Polymere eingebaut.

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt die Patentanmeldung WO 95/26209, Seite 28, Zeile 9 bis Seite 36, Zeile 8. Besagte Passage ist somit Bestandteil dieser Erfindung.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetrafluorethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DAC-RON® oder KODEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt zwischen 75 und 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100

Denier (Gramm pro 9.000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10.000 Meter). Die Form ist nicht besonders eingeschränkt und Beispiele schließen gewebeartige, schmale zylinderartige, geschnitten-/spaltgamartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner als 90° ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die vernetzten quellbaren hydrogelbildenden Polymere am stärksten hydrophil ist. Im Herstellungsprozess wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie beispielsweise Polyethylenterephthalat (DACRON®), und hydrophiles Nylon (HYDROFIL®). Geeignete hydrophile Fasern können auch erhalten werden durch Hydrophilierung hydrophober Fasern, wie beispielsweise die Behandlung thermoplastischer Fasern, erhalten aus Polyolefinen (beispielsweise Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die vernetzten quellbaren hydrogelbildenden Polymerpartikel werden in das beschriebene Fasermaterial eingebettet. Dies kann auf vielfältige Weise geschehen, indem man beispielsweise mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser: (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymener® 557 H, Hercules, Inc. Wilmington Delaware, USA), Polyacrylamid- Überzüge (beschrieben in US-3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann beispielsweise die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens zwei Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, US-3,224,926, US-3,440,135, US-3,932,209, US-4,035,147, US-4,822,453, US-4,888,093, US-4,898,642 und US-5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie beispielsweise Verschmelzen durch Wärmebehandlung, Zugabe von Schmelzklebern, Latexbindern usw. miteinander verbunden.

Beispiele für Verfahren, mit denen man eine absorbierende Zusammensetzung erhält, die beispielsweise aus einem Trägermaterial bestehen, an den ein- oder beidseitig vernetzte quellbare hydrogelbildende Polymere fixiert sind, sind bekannt und von der Erfindung mit eingeschlossen, jedoch nicht limitiert darauf.

Beispiele für Verfahren, mit denen man eine absorbierende Zusammensetzung erhält, die beispielsweise aus in ein Fasermaterial-Gemisch aus synthetischen Fasern (a) und Cellulosefasern (b) eingebetteten vernetzten quellbaren hydrogelbildenden Polymere (c) besteht, wobei das Mischungsverhältnis von (100 bis 0) synthetische Faser: (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so dass eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich von 60 bis 230°C, bevorzugt zwischen 100 und 200°C, besonders bevorzugt zwischen 100 und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunden bis 3 Minuten, bevorzugt 1 Sekunde bis 1 Minute.

Die absorbierende Zusammensetzung wird im allgemeinen beispielsweise mit einer für Flüssigkeit durchlässien Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in der WO 95/26209.

Gegenstand der vorliegenden Erfindung sind weiterhin Hygieneartikel, enthaltend vernetzte quellbare hydrogelbildende Polymere, die mindestens ein hydrophiles Monomer in einpolymerisierter Form enthalten und vernetzt sind mit einem (Meth)acrylsäureeester eines monoalkoxilierten Polyols der Formel (I).

Das erfindungsgemäße Verfahren wird durch die nachfolgenden Beispiele näher erläutert.

Beispiele:

Alle Mengenangaben sind, soweit nicht anders angegeben, Gewichtsteile.

Beispiel 1

87 g Isopropylidentrimethylotpropan werden mit 2,4 g KOH, 45 % in Wasser, in einem Autoklaven vorgelegt und zusammen bei 80°C und reduziertem Druck (ca. 20 mbar) entwässert. Dann werden bei 145 bis 155°C 440 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 Minuten bei ca. 150 °C nachgerührt. Nach Spülen mit Inertgas und Abkühlen auf 60 °C wird der Katalysator durch Zugabe von Natriumpyrophosphat und anschließende Filtration abgetrennt.

Beispiel 2

Es wird verfahren wie unter Beispiel 1. Eingesetzt werden 66 g Isopropylidenglycerin.

Beispiel 3

917 Teile ca. 20fach ethoxiliertes Isopropylidentrimethylolpropan (gemäß Beispiel 1) wird mit 150 Teilen Wasser und 5 Teilen Schwefelsäure hydrolysiert und mit 216 Teilen Acrylsäure in 345 Teilen Methylcyclohexan verestert. Als Hilfsstoffe werden 3 Teile Hydrochinonmonomethylether, 1 Teil Triphenylphosphit und 1 Teil hypophosphoriger Säure zugesetzt. Es werden 29 Teile Wasser abgetrennt bevor das Schleppmittel durch Vakuumdestillation entfernt wird. Das Produkt wird über K300-Filter gereinigt.

Beispiel 4

Es wird verfahren wie unter Beispiel 3. Eingesetzt werden 880 Teile ca. 20fach ethoxiliertes Isopropylidenglycerin (gemäß Beispiel 2).

Vergleichsbeispiel

In eine Petrischale aus Glas mit einer Randhöhe von ca. 10 cm und einem Durchmesser von 18,5 cm wird eine in einem separaten Gefäß hergestellte Lösung überführt. Die Lösung setzt sich aus folgenden Komponenten zusammen: 440 g Wasser, 91,5 g Acrylsäure, 961,1 g einer 37,3 Gew.%-igen Natriumacrylatlösung, 3,66 g Sartomer® SR 9035 (ca. 15fach ethoxiliertes Trimethylolpropan-triacrylat der Firma Sartomer), 0,256 g Natriumpersulfat, 0,098 g DAROCURE® 1173 und 0,049 g IRGACURE® 651 (beides Photoinitiatoren der Firma CIBA GEIGY). Die Glasschale wird in eine Beleuchtungsbox mittig unter eine UV-Lampe UVASPOT L 400 T (der Firma Dr. Hönle GmbH) gestellt. Die Schale wird so platziert, dass der Abstand zwischen Lampe und Flüssigkeitsoberfläche 20 cm beträgt, wodurch eine UVA-Bestrahlungsstärke von ca. 30 $mW/cm^2$ erzielt wird. Unter diesen Bedingungen wird für 12 Minuten bestrahlt. Infolge Polymerisationsreaktion (Temperaturanstieg auf 80-90°C) erhält man einen ca. 5,5 cm dicken Gel-Zylinder, der mechanisch mittels eines Fleischwolfs zerkleinert, bei 160° im Umfufttrockenschrank getrocknet, mit einer Ultrazentrifugalmühle gemahlen wird. Anschließend wird die Siebfraktion von 150-800 μm isoliert.

Beispiel 5

Es wird verfahren wie unter dem Vergleichsbeispiel. Eingesetzt werden 4,43 Teile ca. 20fach monoethoxyliertes Trimethylolpropan-triacrylat (gemäß Beispiel 3).

Beispiel 6

Es wird verfahren wie unter dem Vergleichsbeispiel. Eingesetzt werden 4,28 Teile ca. 20fach monoethoxyliertes Glycerin-triacrylat (gemäß Beispiel 4).

Testmethoden

Zur Bestimmung der Güte der Oberflächenvernetzung kann das getrocknete Hydrogel mit folgenden Testmethoden untersucht werden.

Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0,2000 ± 0,0050 g getrocknetes Hydrogel (Kornfraktion 106 - 850 μm) in einem 60 x 85 mm großen Teebeutel eingewogen, der anschließend verschweißt wird. Der Teebeutel wird für 30 Minuten in einen Überschuss von 0,9 gew.-%iger Kochsalzlösung gegeben (mindestens 0,83 l Kochsalzlösung/1 g Polymerpulver). Anschließend wird der Teebeutel 3 Minuten lang bei 250 G zentrifugiert. Die Bestimmung der vom Hydrogel festgehaltenen Flüssigkeitsmenge geschieht durch Auswägen des zentrifugierten Teebeutels.

Absorption unter Druck (AUL Absorbency Under Load) 0,3 psi (2070 Pa)

Die Messzelle zur Bestimmung der AUL 0,3 psi ist ein Plexiglas-Zylinder mit einem Innendurchmesser von 60 mm und einer Höhe von 50 mm, der an der Unterseite einen angeklebten Edelstahl-Siebboden mit einer Maschenweite von 36 μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 576 g. Zur Durchführung der Bestimmung der AUL 0,3 psi wird das Gewicht des leeren Plexiglas-Zylinders und der Plastikplatte ermittelt und als $W_0$ notiert. Dann werden 0,900 ± 0,005 g Hydrogel-formendes Polymer (Korngrößenverteilung 150 - 800 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 120 mm und einer Höhe von 30 mm wird eine keramische Filterplatte mit einem Durchmesser von 120 mm und einer Porosität 0 gelegt und soviel 0,9 gew.-%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$AUL\ 0{,}3\ psi\ [g/g] = [W_b - W_a]/[W_a - W_0]$$

Extrahierbare Anteile

Die Bestimmung des nach 16 Stunden extrahierbaren Anteils (Extractables 16h) erfolgte analog wie in EP-A-0 811 636, Seite 13, Zeilen 1 bis 19 beschrieben.

Die erzielten Eigenschaften der Hydrogele sind in nachfolgender Tabelle zusammengefasst:

| | CRC [g/g] | AUL (0,3psi) | Extractables [%] |
|---|---|---|---|
| Vergleichsbeispiel | 42,0 | 7,9 | 15,3 |
| Beispiel 5 | 38,6 | 6,9 | 12,6 |
| Beispiel 6 | 38,8 | 6,1 | 13,4 |

Für den Fachmann ist leicht zu erkennn, dass die erfindungsgemäßen Vernetzer bei equimolarer Einsatzmenge besser vernetzen, was sich insbesondere an den niedrigen extrahierbaren Anteilen zeigt.

**Patentansprüche**

**1.** (Meth)acrylsäureester monoalkoxilierter Polyole der allgemeinen Formel I

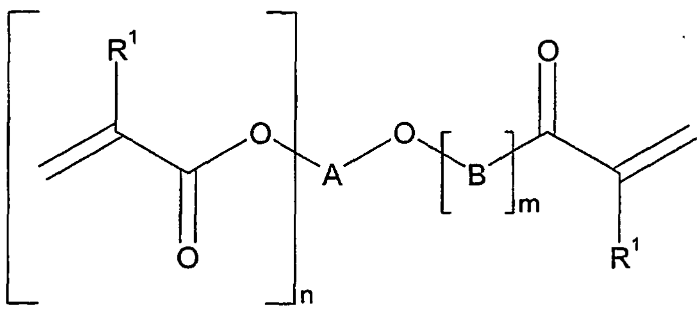

(I)

in der

$R^1$ Wasserstoff oder Methyl,
n eine ganze Zahl von 2 bis 5,
m eine ganze Zahl von 1 bis 100,
A $C_3$- bis $C_{20}$-Alk(n+1)yl oder $C_3$- bis $C_{20}$-Heteroalk (n+1) yl

bedeuten, und
B für gleiche oder verschiedene Reste, ausgewählt aus der Gruppe

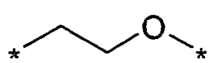

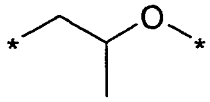

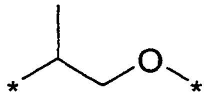

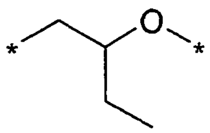

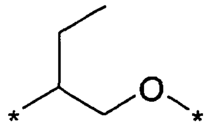

steht und mit * die Verknüpfungspositionen **gekennzeichnet** sind.

**2.** (Meth)acrylsäureester monoalkoxilierter Polyole der allgemeinen Formel I gemäß Anspruch 1, in der

$R^1$ Wasserstoff oder Methyl,
n 2 oder 3,
m eine ganze Zahl von 2 bis 50,
A $C_3$- bis $C_{10}$-Alk (n+1) yl

bedeuten, und
B für gleiche oder verschiedene Reste, ausgewählt aus der Gruppe

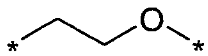

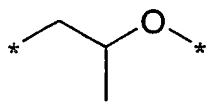

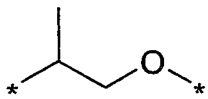

steht und mit * die Verknüpfungspositionen **gekennzeichnet** sind.

**3.** (Meth)acrylsäureester monoalkoxilierter Polyole der allgemeinen Formel I gemäß Anspruch 1 in der

$R^1$ Wasserstoff oder Methyl,
n 2,
m eine ganze Zahl von 3 bis 30,
A $C_3$- bis $C_6$-Alk (n+1) yl

bedeuten, und
B

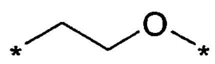

ist und mit * die Verknüpfungspositionen **gekennzeichnet** sind.

**4.** (Meth)acrylsäureester monoalkoxilierter Polyole der allgemeinen Formel I gemäß einem der Ansprüche 1 bis 3 in der das Polyol Glycerin ist.

**5.** Verfahren zur Herstellung der (Meth)acrylsäureester monoalkoxilierter Polyole gemäß einem der Ansprüche 1 bis 4 umfassend die Schritte

a) Hydrolyse des partiell geschützten monoalkoxilierten Polyols in Anwesenheit mindestens eines Hydrolysekatalysators und Wassers,
b) Umsetzung des monoalkoxilierten Polyols mit (Meth)acrylsäure in Anwesenheit mindestens eines Veresterungskatalysators und mindestens eines Polymerisationsinhibitors sowie gegebenenfalls eines mit Wasser ein Azeotrop bildenden Lösungsmittels unter Bildung des (Meth)acrylsäureesters des monoalkoxlierten Polyols, wobei b) im selben Reaktor wie a) erfolgen kann,
c) gegebenenfalls Entfernen zumindest eines Teils des in b) entstehenden Wassers aus dem Reaktionsgemisch, wobei c) während und/oder nach b) erfolgen kann,
d) gegebenenfalls Neutralisation des Reaktionsgemischs,
e) falls ein Lösungsmittel eingesetzt wurde gegebenenfalls Entfernen dieses Lösungsmittels.

**6.** Quellbares hydrogelbildendes Polymer, enthaltend einen einpolymerisierten Innenvernetzer der allgemeinen Formel I nach Anspruch 1 bis 4.

**7.** Verfahren zur Herstellung vernetzter quellbarer hydrogelbildender Polymere nach Anspruch 6, **dadurch gekennzeichnet, dass** man eine wäßrige Mischung, enthaltend ein hydrophiles Monomer, gegebenenfalls mindestens eine weitere monoethylenisch ungesättigte Verbindung, mindestens einen (Meth)acrylsäureester monoalkoxilierter Polyole, mindestens einen Radikalstarter sowie gegebenenfalls mindestens eine Pfropfgrundlage, polymerisiert und gegebenenfalls das erhaltene Reaktionsgemisch nachvernetzt, trocknet und auf die gewünschte Komgröße bringt.

**8.** Verwendung von vernetzten quellbaren hydrogelbildenden Polymeren nach Anspruch 6 zur Herstellung eines Hygieneartikels.

**9.** Hygieneartikel, enthaltend ein vernetztes quellbares hydrogelbildendes Polymer nach Anspruch 6.

## Claims

**1.** (Meth)acrylic esters of monoalkoxylated polyols of the general formula I

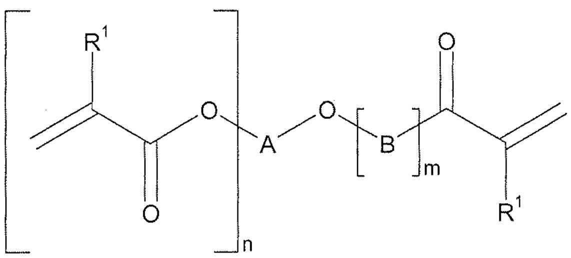

(I)

where

$R^1$ is hydrogen or methyl,
n is an integer from 2 to 5,
m is an integer from 1 to 100,
A is $C_3$ to $C_{20}$ alk(n+1)yl or $C_3$ to $C_{20}$ heteroalk(n+1)yl, and

B represents identical or different radicals selected from the group consisting of

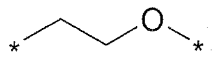

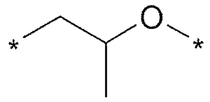

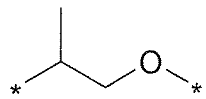

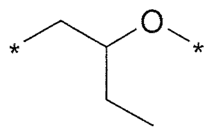

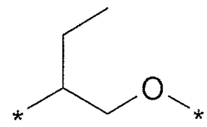

where * identifies the positions of attachment.

**2.** (Meth)acrylic esters of monoalkoxylated polyols of the general formula I according to claim 1 where

$R^1$ is hydrogen or methyl,
n 2 or 3,
m is an integer from 2 to 50,
A $C_3$ to $C_{10}$ alk(n+1)yl, and

B represents identical or different radicals selected from the group consisting of

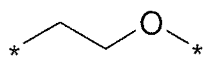

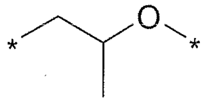

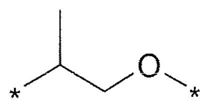

where * identifies the positions of attachment.

**3.** (Meth)acrylic esters of monoalkoxylated polyols of the general formula I according to claim 1 where

$R^1$ is hydrogen or methyl,
n is 2,
m is an integer from 3 to 30,
A is $C_3$ to $C_6$ alk(n+1)yl, and
B is

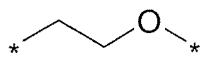

where * identifies the positions of attachment.

**4.** (Meth)acrylic esters of monoalkoxylated polyols of the general formula I according to any of claims 1 to 3 in which formula the polyol is glycerol.

**5.** A process for preparing the (meth)acrylic esters of monoalkoxylated polyols according to any of claims 1 to 4, comprising the steps of

a) hydrolyzing the partially protected monoalkoxylated polyol in the presence of at least one hydrolysis catalyst and water,
b) reacting the monoalkoxylated polyol with (meth)acrylic acid in the presence of at least one esterification catalyst and of at least one polymerization inhibitor and if appropriate of a water-azeotroping solvent to form the (meth)acrylic ester of the monoalkoxylated polyol, it being possible to carry out b) in the same reactor as a),
c) if appropriate removing from the reaction mixture at least a portion of the water formed in b), it being possible to carry out c) during and/or after b),
d) if appropriate neutralizing the reaction mixture,
e) when a solvent was used, if appropriate removing this solvent.

**6.** Swellable hydrogel-forming polymer comprising a copolymerized internal crosslinker of the general formula I according to any of claims 1 to 4.

**7.** A process for preparing crosslinked swellable hydrogel-forming polymers according to claim 6, which comprises polymerizing an aqueous mixture comprising a hydrophilic monomer, if appropriate at least one further monoethylenically unsaturated compound, at least one (meth)acrylic ester of monoalkoxylated polyols, at least one free-radical initiator and if appropriate also at least one grafting base, and if appropriate the reaction mixture obtained being postcrosslinked, dried and brought to the desired particle size.

**8.** The use of crosslinked swellable hydrogel-forming polymers according to claim 6 for manufacturing a hygiene article.

**9.** A hygiene article comprising a crosslinked swellable hydrogel-forming polymer according to claim 6.

## Revendications

**1.** Esters d’acide (méth)acrylique de polyols monoalcoxylés de formule générale I

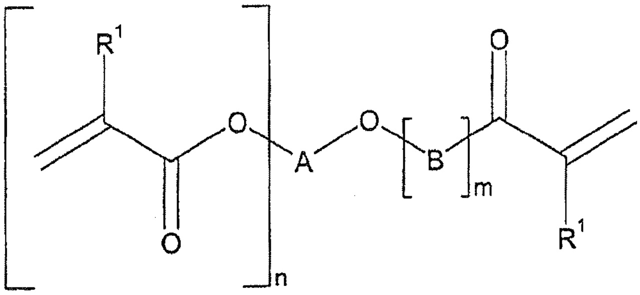

(I)

dans laquelle

$R^1$ représente de l'hydrogène ou du méthyle,
n représente un nombre entier de 2 à 5,
m représente un nombre entier de 1 à 100,
A représente de l'alk(n+1)yle en $C_3$ à $C_{20}$ ou de l'hétéroalk(n+1)yle en $C_3$ à $C_{20}$, et
B représente des radicaux semblables ou différents, choisis parmi les groupes

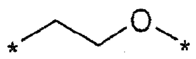

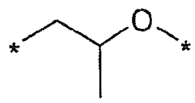

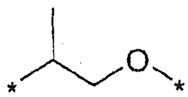

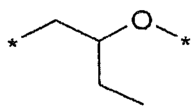

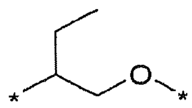

et les positions de connexion sont marquées par un *.

**2.** Esters d'acide (méth)acrylique de polyols monoalcoxylés de formule générale I selon la revendication 1, dans laquelle

$R^1$ représente de l'hydrogène ou du méthyle,
n signifie 2 ou 3,
m représente un nombre entier de 2 à 50,
A représente de l'alk(n+1)yle en $C_3$ à $C_{10}$, et
B représente des radicaux semblables ou différents, choisis parmi les groupes

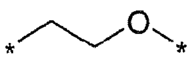

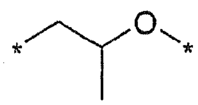 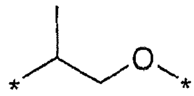

et les positions de connexion sont marquées par un *.

**3.** Esters d'acide (méth)acrylique de polyols monoalcoxylés de formule générale I selon la revendication 1, dans laquelle

$R^1$ représente de l'hydrogène ou du méthyle,
n signifie 2,
m représente un nombre entier de 3 à 30,
A représente de l'alk(n+1)yle en $C_3$ à $C_6$,

et
B est

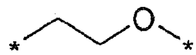

et les positions de connexion sont marquées par un *.

**4.** Esters d'acide (méth)acrylique de polyols monoalcoxylés de formule générale I selon l'une quelconque des revendications 1 à 3, dans laquelle le polyol est de la glycérine.

**5.** Procédé de préparation des esters d'acide (méth)acrylique de polyols monoalcoxylés selon l'une quelconque des revendications 1 à 4, comportant les étapes

a) hydrolyse du polyol monoalcoxylé partiellement protégé en présence d'au moins un catalyseur d'hydrolyse et d'eau,
b) réaction du polyol monoalcoxylé avec de l'acide (méth)acrylique en présence d'au moins un catalyseur d'estérification et d'au moins un inhibiteur de polymérisation ainsi que le cas échéant un solvant formant avec de l'eau un azéotrope, avec formation de l'ester d'acide (méth)acrylique du polyol monoalcoxylé, b) pouvant avoir lieu dans le même réacteur que a),
c) le cas échéant élimination dans le mélange réactionnel d'au moins une partie de l'eau provenant de b), c) pouvant avoir lieu pendant et/ou après b),
d) le cas échéant neutralisation du mélange réactionnel,
e) dans le cas où un solvant a été utilisé, élimination le cas échéant de ce solvant.

**6.** Polymère gonflable formant un hydrogel, contenant un agent de réticulation intérieur à l'état polymérisé de formule générale I selon les revendications 1 à 4.

**7.** Procédé de préparation de polymères gonflables réticulés formant un hydrogel selon la revendication 6, **caractérisé en ce qu'**un mélange aqueux, contenant un monomère hydrophile, le cas échéant au moins un autre composé monoéthyléniquement insaturé, au moins un ester d'acide (méth)acrylique de polyols monoalcoxylés, au moins un amorceur de radical ainsi que le cas échéant au moins une base de greffe, est polymérisé et le mélange réactionnel obtenu est le cas échéant post-réticulé, séché et amené à la grosseur de grain souhaitée.

**8.** Utilisation de polymères gonflables réticulés formant un hydrogel selon la revendication 6 pour la fabrication d'un article hygiénique.

**9.** Article hygiénique, contenant un polymère gonflable réticulé formant un hydrogel selon la revendication 6.